# EUROPEAN PATENT APPLICATION

(11) **EP 0 628 307 A2**
(43) Date of publication of application: **14.12.1994**
(21) Application number: 94303655.8
(22) Date of filing: 23.05.1994
(51) Int. Cl.: A61K 9/16

(54) **Methods for administering biological agents and microparticle compositions useful in these and other methods**

(30) Priority: 27.05.1993 US 68413; 16.12.1993 US 168941
(71) Applicant: ELI LILLY AND COMPANY, Indianapolis, Indiana 46285 (US)
(72) Inventor: Craft, Libbey Sue, Indianapolis, Indiana 46236 (US); Ferguson, Thomas Harry, Greenfield, Indiana 46140 (US); Heiman, Mark Louis, Indianapolis, Indiana 46250 (US); Thompson, William Webster, Indianapolis, Indiana 46226-1542 (US)
(74) Representative: Tapping, Kenneth George

(57) **Abstract**

A method for administering biological agents to eggs is disclosed which comprises providing the agents in a particulate carrier and injecting the carrier into the air cells of the eggs. The egg is preferably maintained in a vertical position with the air cell on top to facilitate migration of the particulate carrier between the inner and outer membranes which define the air cell, to the lower end of the egg. The particulate carrier releases the biological agent to the surrounding fluid and blood vessels. In addition, the carrier is embodied in the bird upon hatching from the egg, and therefore is available to continue to release the biological agent to the bird posthatch.

Also disclosed is a composition of polyester microparticles containing bioactive polypeptide agents and methods for preparing the composition and administering bioactive agents. The composition comprises biocompatible, biodegradable microparticles having a polyester matrix and from about 5% to about 25% by weight of a biologically active, water-soluble polypeptide dispersed throughout the matrix, the polypeptide selected fom the group consisting of growth hormone releasing factor, synthetic analogs of growth hormone releasing factor, and pharmacologically active fragments thereof. The method for preparing the composition includes dissolving polyester in an organic solvent; suspending a biologically active agent in the polyester solution; emulsifying the suspension into an aqueous medium in which the agent is insoluble and evaporating the solvent from the emulsion to produce microparticles. The method for administering a bioactive agent to an organism involves suspending the microparticles in a suitable liquid and injecting the organism.

## Description

The present invention relates to the field of methods for administering biological agents to birds, and particularly to administering agents to an egg prior to hatching. The present invention also relates to microparticle compositions useful in practicing the foregoing methods and in other methods of delivering materials to animals, as well as to processes for preparing such compositions.

The desirability of injecting eggs has been recognized for some time. Initially, the purpose of injecting eggs was to prepare various vaccines using the egg as a growth medium for the vaccine. The vaccine was then harvested from the egg and used as desired. Egg injections have also been used in toxicology studies. More recently, a purpose has been to inject eggs for accomplishing some beneficial or therapeutic affect on the bird that will eventually hatch from the egg. One advantage of injecting the egg rather than the live bird is related to the ease of injection. Eggs can be kept immobile and handled rather efficiently in comparison to newborn or older birds. Furthermore, in addition to the mechanical ease of injecting eggs, there also appear to be certain therapeutic advantages in inoculating or otherwise treating embryos rather than live birds. These advantages have become particularly important in the poultry industry, e.g., for chickens and turkeys.

Given the desirability of injecting eggs for the described and other purposes, several basic techniques have been attempted. These generally include either forcing fluids through the shell of an egg using some sort of pressurization system, or physically forming an opening in the shell of an egg and then adding the desired fluid. Injection using some type of needle arrangement has been one of the basic techniques for physically opening an egg for such purposes.

In the past, the desired fluids have been administered either to the air cell of the egg or directly into the yolk sac or amniotic fluid. Fluids delivered into the air cell simply pass through the inner membrane of the egg into nearby blood vessels. This approach has the advantage of not requiring that instruments be passed through the inner membrane, which would require sterile procedures and instruments. Delivery into the amniotic fluid also raises the risk that the injection device, commonly a needle, may injure or even destroy the live embryo.

A further potential problem with administration of agents to a developing embryo relates to timing. Certain agents are optimally delivered at a particular stage of development. However, embryos may develop at different rates. Thus, administration for one egg may preferably occur on day 17 of development, whereas the timing for other eggs may be day 16 or day 18. However, it is impractical to individually monitor eggs when a large number are desired to be treated. Also, some agents may desirably be administered over a period of time, requiring to date repeated administrations.

A need has therefore existed for a method of administering biological agents to eggs which addresses the various issues above presented. For example, the method would advantageously be simple and avoid the need for sterile technique. Further, the technique would preferably avoid the problem of different development rates of embryos, and would circumvent the need to make repeated administrations of the agent. The present invention satisfies the foregoing needs and provides advantages not available from prior art methods.

As noted, the present invention is also directed to microparticle compositions useful in practicing the foregoing methods and in other methods of delivering materials to animals.

Microparticles are spherical polymeric particles ranging in size from greater than one micron up to 2000 microns. Microparticles include microcapsules in which the biological agent is uniformly confined within a cavity, and microspheres in which the agent is dispersed throughout the microparticle. Many processes can be used for the preparation of microparticles, including solvent evaporation, organic phase separation, interfacial polymerization, emulsion polymerization, and spray drying. However, only a few methods are acceptable for preparing peptide microparticles. The physicochemical properties of many peptides make their formulation difficult, and inactivation is possible during their incorporation into microparticles.

Numerous polymers have been used as matrices for microparticles, including polysaccharides, polyesters and nonbiodegradable synthetic polymers. Most methods for microencapsulation of peptides employ polyesters, especially poly(D,L-lactide-co-glycolide). The polyesters are desirable for this purpose because they are biodegradable or bioerodible, readily available, easily processed and non-toxic.

Microspheres of lactic and glycolic acids may be obtained conveniently by a solvent evaporation process which is, in spite of some limitations, compatible with the handling of numerous peptides. However, a major obstacle to the particulate formulation of peptides is the high water solubility of those molecules. The typical processes for microencapsulation are based on the affinity of the compound for the polymer or for the lipophilic phase of an emulsion. As a result, drug loadings for peptides have typically been less than 10% with the solvent evaporation processes used in the past.

Prior art efforts to overcome these problems have thus far included the use of a double emulsion technique, and of a phase separation process induced by the addition of a silicone oil. However, there has remained a need for methods for the preparation of biodegradable microparticles incorporating peptides which are stable and active, and which pay out at desired rates over time.

Briefly describing one aspect of the present invention, there is provided a method for administering a biological agent to an egg which comprises delivering a particulate carrier containing the agent into the air cell of the egg. The particulate carrier migrates between the inner and outer membranes (which define the air cell at the rounded end) to a position away from the air cell. Advantageously, the biological agent is released from the carrier over a period of time. Moreover, the particulate carrier is incorporated into the bird after hatching, thus providing for continuing administration of the biological agent to the young bird.

It is an object of the present invention to provide a method for administering a biological agent to an egg, and further to a developing young bird. A further object of the present invention is to provide a method for administering a biological agent which method is readily performed, does not require sterile technique, and minimizes the risk of injury to the developing embryo within the egg. It is another object of the present invention to provide a method which can deliver a biological agent to the developing embryo over a prolonged period of time, and to the young bird resulting therefrom.

Further objects and advantages of the present invention will be apparent from the description of the preferred embodiment which follows.

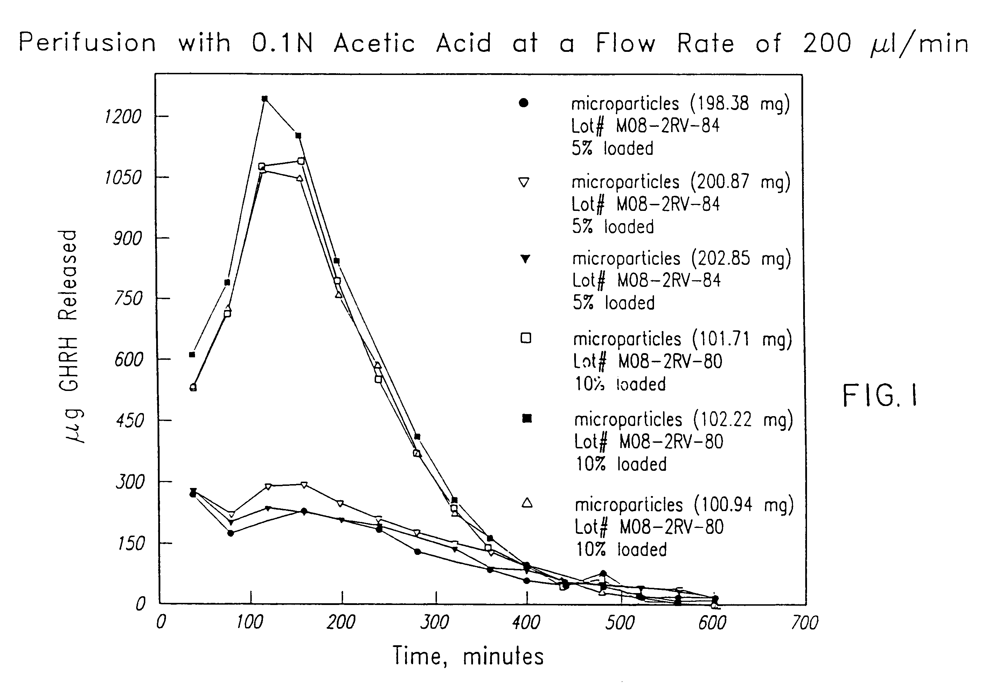

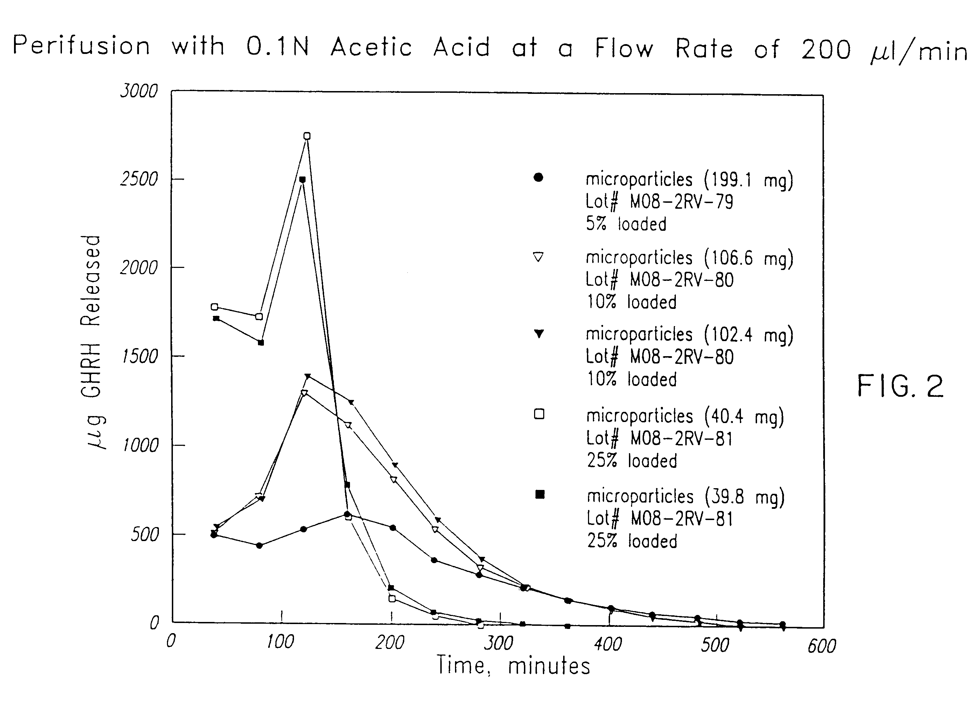

FIGS. 1 and 2 are graphs representing in vitro studies demonstrating the rate of release of GHRH from microspheres loaded therewith.

For the purposes of promoting an understanding of the principles of the invention, reference will now be made to the preferred embodiment and specific language will be used to describe the same. It will nevertheless be understood that no limitation of the scope of the invention is thereby intended, such alterations, modifications and further applications of the principles of the invention being contemplated as would normally occur to one skilled in the art to which the invention relates.

The present invention provides an advantageous method for administering biological agents. A particulate carrier is provided which includes one or more biological agents in a form which will be delivered to a developing embryo and the young bird resulting therefrom. There are two main shell membranes in the avian egg. Only a thin layer of albumen separates these inner and outer membranes which are adjacent, except at the broad end where they separate to form the air cell. The method of the present invention generally includes the placement of the particulate carrier into the air cell of an egg. Surprisingly, it has been found that the particulate carrier will migrate from the air cell, between the inner and outer membranes, to the opposite end of the egg.

The particulate carrier and biological agent may be selected to provide a prolonged release of the agent over time. As the embryo develops into the young bird, the particulate carrier is embodied within the bird. Therefore the carrier is available to deliver biological agent to the egg, and even to the bird after it hatches from the egg. The prolonged release avoids the problem of having to meet a specific time for administration of the biological agent. For example, if the target time for delivery of the agent ranges from embryonic day 16 to day 18 (E16-E18), depending on the rate of development of the embryo, then the agent may be administered in accordance with the present invention on day E15, and the time release of the agent over a period of several days will ensure that the agent is available at the desired time. The invention similarly addresses the problem where an agent may need to be present for a particularly long period during fetal development, especially during the later days when the fetus is more sensitive to manipulation.

As is well known, avian eggs include an inner membrane and an outer membrane, with an air cell defined therebetween at one end. The outer membrane typically is just below a relatively hard shell, which protects the embryo during development. The inner membrane also provides a measure of protection for the embryo, since it screens out many harmful substances, such as bacteria, which could otherwise infect the embryo. The present method involves the placement of a particulate carrier into the air cell between the inner and outer membranes of the egg.

Administration of the particulate carrier into the air cell provides significant advantages. Methods and instruments are readily available for placing the particulate carrier into this position within an egg. In addition, the criticality of placement is reduced, since the air cell is readily located, and placement such as by injection can be reliably and safely performed. Since the inner membrane of the egg is not breached, the need for sterile technique is avoided. Risk of injury to the embryo also is not presented.

Various techniques are known for delivering a material into the air cell of an egg. The method is not critical, except that it is preferred that minimum disruption to the egg occur. Direct injection into the air cell is preferred. Typically, a hypodermic syringe fitted with a needle, typically of about 18 to 22 gauge, is suitable for this purpose. To inject into the air cell, the needle need only be inserted into the egg a few millimeters. A pilot hole may be punched or drilled through the shell prior to insertion of the needle to avoid damaging or dulling the needle. If desired, the hole in the egg can be resealed with a suitable material, such as a glue, wax or the like, or may be left open.

An advantage of the present invention is that it is well adapted to use with high speed, automated injection systems. Numerous such systems are now available, including those described in United States Patent No. 5,136,979, issued to Paul et al.; United States Patent No. 5,056,464, issued to Lewis et al.; United States Patent Nos. 4,681,063 and 4,903,635, issued to Hebrank; and United States Patent Nos. 4,040,388, 4,469,047 and 4,593,646, issued to Miller; all assigned to Embrex, Inc. of North Carolina. The disclosures of the foregoing patents are hereby incorporated by reference. These devices provide for the administration of materials into the air cells of eggs at a relatively rapid rate. In addition, available devices, such as the one described in the Paul et al. patent, can accommodate eggs of different sizes, shapes and orientation. An Embrex, Inc. automated injection system, marketed under the name INOVOJECT, has been used with good results.

The present invention is useful for the delivery of a wide variety of biologically active agents to birds or bird eggs. The term "bird" is intended to include any avian species, especially poultry which are commercially raised for eggs or meat. Accordingly, the term bird is particularly intended to encompass hens, cocks and drakes of chickens, turkeys, ducks, geese, quail and pheasant.

The agents to be administered include any which may be desired to be administered to an embryo or young bird, and which are compatible with and releasable from the particulate carrier employed. Biological agents may include neuropeptides, peptide mimicks, antigens, genes (DNA or RNA), enzymes, hormones, antibiotics and various other biochemical substances, which may be given individually or in combination. In addition, agents may include those for which research studies may be desired, such as for testing of compounds in toxicity studies. Since the present invention operates essentially as a physical phenomenon, i.e., placement and migration of the particulate carrier, it will be appreciated that the selected biological agent is not critical.

Such biological agents are well known in the art, and are recited in numerous articles and patents. For example, United States Patent No. 5,106,617, issued to Federicksen et al. and incorporated herein by reference, describes the utility of administering a T-cell growth factor comprising Interleukin-2 when administered in ovo on about the eighteenth day of incubation. Federicksen also discloses the concurrent administration of a vaccine in ovo, including both live vaccines, such as turkey herpes virus vaccine and the Bursal Disease Vaccine, Lukert strain, and non-replicating vaccines such as killed viruses, peptides, proteins and anti-idiotipic antibodies. In PCT Application No. WO91/12016, published August 22, 1991 and hereby incorporated by reference, there is reported the administration of physiologically active peptides. Exemplary pituitary peptides include growth hormone, thyrotropin, alphamelanocyte stimulating hormone, prolactin, luteinizing hormone, adrenocorticotropin, and beta-endorphin. Exemplary hypothalamic releasing hormones include thyrotropin releasing hormone (TRH), gonadotropin releasing hormone, somatostatin, corticotropin releasing hormone, and growth hormone releasing hormone. Exemplary gastrointestinal peptides include vasoactive intestinal polypeptide, cholecystokinin, gastrin, substance P, neurotensin, glucagon, bombesin, secretin and motilin.

Various other agents are known, and still more are being identified on a continuing basis. In U.S. Patent No. 4,917,045, issued to Wiegland, there is described the use of physiologically acceptable organic silicon compounds or silicic acid to stimulate the growth of bone tissue. Sensitizing the immune system can be accomplished by in ovo injection of dinitrophenylated keyhole limpet hemocyanine (DNP-KLH) or dextran (DNP-D). "Elicitation of delayed type hypersensitivity in chicks after in ovo sensitization with different molecular forms of the same hapten," O. Moriya; Y. Ichikawa, Microbiol. Immunol.; 27(9) pp. 779-785 (1983). In ovo administration of ovine growth hormone (oGH) in isotonic salt buffered to pH 8.3 with NaHCO₃, given to chickens on day E11, has been shown to increase body weights, skeletal growth and feed efficiencies. "In Ovo Growth Hormone Alters Growth and Adipose Tissue Development of Chickens," P.S. Hargis, S.L. Pardue, A.M. Lee, and G.W. Sandel, Growth, Development & Aging, 1989, 53, pp. 93-99.

Numerous other reports describing substances desired to be presented in ovo exist in the literature. See, for example, "Effects of Diazinon on the Anatomical and Embryological changes in the Developing Chick Embryo," J.H. Cho, C.E. Lee., Res. Rep. Rural Dev. Adm. (Suweon), 32 (3 Vet.) 1990, pp. 35-47 (diazinon on day E3); "Bilirubin and Heme as Growth Inhibitors of Chicken Embryos In Ovo," R. Vassilopoulou-Sellin, P. Foster, C.O. Oyedeji, Pediatr. Res., 27(6) 1990, pp. 617-621 (bilirubin and heme); "Boiler Safety Studies of Marek's Vaccination In Ovo Using the Inovoject," C.M. Hoyle, R.P. Gildersleeve, Poult. Sci., 69 (Suppl. 1) 1990, p. 65 (Marek's disease vaccine); "Post-Natal Development of Male and Female Broilers After One or Several Thyrotropin Releasing Hormone TRH Injections During Egg Incubation," J.C. Blum, M.R. Salichon, J.P. Vigneron, Arch. Geflueaelkd, 53(6), 1989, pp. 265-268 (thyrotropin releasing hormone); "Increased Hatchability of Turkey Eggs by Biotin Egg Injection," E.J. Robel, V.L. Christensen, Poult. Sci., 65 (Suppl. 1) 1986, p. 112 (biotin). The disclosures of all of the foregoing articles are hereby incorporated herein by reference.

The biological agent is contained by a suitable, biocompatible (physiologically acceptable) particulate carrier. The carrier may be any of a variety of particulate materials, referred to herein generally as "microparticles," such as microspheres, lipid vesicles, or modified forms thereof. Microspheres are typically made of silica, glass, various biodegradable, high polymers (e.g., polylactide and polyglycolide polymers and copolymers thereof, polyhydroxybutyric acid, cellulosics, starch, carbohydrate gums, etc.) or proteins (e.g., gelatin, albumen). Lipid vesicles (e.g., liposomes) appear to be ideal delivery vehicles for injections into eggs since they can be tailored to a desired application and biological agent, e.g., made with different charge (negative, positive, neutral); they can be made fusagenic to egg membranes through proper selection of the phospholipid; can carry both water soluble and insoluble compounds; and use natural or natural appearing excipients. Liposomes may be either phospholipid or non-phospholipid. Preferred non-phospholipid liposome vehicles are the paucilamellar liposomes produced by Micro Vesicular Systems, Inc., and called "Novasome" vesicles, which have the advantage of minimizing problems with oxidation.

The biological agent is provided in the microparticles by known techniques, such as by incorporation in the particles during formation, absorption into the microparticles, or coating onto the microparticles. Inclusion of the biological agent in any of these fashions provides the agent in a manner such that the agent will be delivered from the microparticles over a period of time. As a result, the particulate carrier, and the manner of containment thereby of the biological agent, can be selected to provide delivery of the agent at a desired time and rate, which may occur during development of the embryo and/or growth of the young bird after hatching.

The particles are preferably at least as dense as the fluid between the membranes. Higher density will simply affect the rate of migration of the particles to the bottom end of the egg. Particles having a density less than the fluid between the membranes could be caused to rise up through the fluid by inverting the eggs, but this is not preferred since it requires an additional step which is not needed with proper selection of the particle density. It would also suffocate the embryo because the air space permits gas exchange.

Sizes of the particulate materials comprising the carrier will vary, depending on the material selected as well as other factors. The microparticles are sized to permit migration of the microparticles between the inner and outer membranes of the egg. As a consequence, the microparticles move out of the air cell to a position spaced from the air cell, which results in the particles being incorporated into the bird upon hatching. The microparticles preferably are sized to freely migrate between the inner and outer membranes of the egg. For example, the microparticles suitably are sized to be not greater in size than the typical distance between the adjacent portions of the inner and outer membranes, preferably not greater than half such distance. Typical sizes are from about 5 to about 500 microns. Preferably, the microparticles range from about 125 to about 250 microns. It will be appreciated that the preferred sizes will vary, depending on the type of microparticle and the distance between the inner and outer egg membranes in different avian species.

A biologically effective amount of agent is introduced into the air cell. It will be appreciated that the amount of agent used will depend upon the agent employed and the effect desired. Factors will include the amount, desired release rate, concentration and duration of administration intended, as well as other considerations well known in the art. Similarly, the amount of particulate carrier will be selected depending on the size of the microparticles, the amount and concentration of agent contained by the microparticles, the release rate of the agent from the microparticles, and other typical factors.

In addition, the time of delivery to the egg of the particulate carrier with biological agent is selected to produce the desired result. The timing will again depend on known factors, such as the agent being administered, developmental stage of the embryo, and the effect desired. These factors are well known in the art, and preferred days of administration for various biological agents have been described in the literature or may be determined without undue experimentation. When a particularly preferred date is desired, such as the sixteenth day of incubation, then the particulate carrier is typically provided a day or two prior to this date. This ensures that the biological agent will be available on the desired date, and also allows for variations in release rate of microparticles and time to achieve peak concentration.

The particulate carrier migrates between the inner and outer membranes to a position distant from the air cell in which the carrier is initially placed. It is preferred that the eggs be oriented vertically, with the air cell at the top, for at least a period of time after delivery of the carrier into the air cell. The time of vertical orientation preferably occurs immediately after injection of the microparticles, and lasts for a time sufficient to promote migration of the microparticles to the bottom end of the egg opposite the air cell. Such time is preferably continuous, although this is not required. As a matter of convenience, the time of administration may be coordinated with the normal handling of the eggs based upon other considerations. For example, it is common to maintain eggs in an incubator for a period of days, typically about 17 days for chickens, during which time the eggs are periodically moved. Oftentimes, the eggs are thereafter moved to a hatcher, during which time the eggs are not appreciably moved. It will be appreciated that the transition from the incubator to the hatcher will frequently be a convenient time to administer the particulate carrier and contained biological agent to the eggs. However, this time period for administration would be selected more for convenience than for necessity, and other times of administration either before or after this time would be acceptable.

Preferred administration dates have been identified for a large number of compounds. The date of administration is selected such that the biological agent is capable of producing a desired physiological response in the embryo or later developing bird. For example, it has been reported that hatchability of fertile turkey eggs is increased by administering an effective amount of exogenous pyridoxine (vitamin B₆) on approximately day E25 of incubation. Physiologically active peptides, including pituitary peptides, hypothalamic releasing hormones, and gastrointestinal peptides, particularly those which elicit an endocrine response, have been reported to be advantageously administered to chickens during the last quarter of in ovo incubation. The desired date of administration for various agents can be selected by those skilled in the art. Preferred dates of administration can be determined for various agents of choice.

The following examples are provided for purposes of illustrating the present invention. However, no limitations on the invention are intended thereby.

To demonstrate the operation of the present invention, microparticles were prepared for testing using DL-lactide-coglycolide (PLA/PGA), a random copolymer of lactic acid and glycolic acid. One PLA/PGA polymer used had a ratio of PLA to PGA of 85:15, an inherent viscosity of 0.58 dL/g, and a molecular weight of approximately 90,900 daltons. This polymer is available from Birmingham Polymers, Incorporated (BPI), and is prepared by ionic, ring-opening, addition polymerization of the respective cyclic dimers of lactic and glycolic acids. Upon exposure to water, this aliphatic polyester degrades through hydrolysis of ester linkages to yield the biocompatible products lactic acid and glycolic acid. The 85:15 copolymer has been reported to have a biodegradation time of approximately 5 months, depending on surface area, porosity and molecular weight. Other blends of PLA/PGA have degradation times ranging from 2 months to 24 months. A 50:50 PLA/PGA polymer was alternately used having a reported degradation time of 2 months.

### EXAMPLE 1

### Preparation of Red/Green Dyed Blank PLA/PGA Microparticles

Blank PLA/PGA microparticles for evaluation in chicken eggs were prepared. The first set of microparticles was dyed using American Cyanamid Company Calco Oil Red N-1700 dye, and the second set of microparticles used Calco Victoria Green Base dye, also from American Cyanamid Company. The preparation procedure for each microparticle run was essentially the same. Three grams of PLA/PGA (85/15), inherent viscosity of 0.58 dL/g in CHCl₃ at 30°C (M_{W} = 90,900 Daltons, M_{N} = 50,100 Daltons), BPI, was dissolved in approximately 40 ml of dichloromethane. A small amount of dye was added to the polymer solution, each dye being oil soluble. The organic phase was emulsified with stirring into 250 ml of deionized water containing 0.25% w/v Air Products V-205 poly(vinyl alcohol). Stirring was allowed to continue for 16 hours at room temperature and atmospheric pressure to ensure complete loss of dichloromethane. Resulting microparticles were recovered using vacuum filtration. Microparticles were dried under vacuum and sized by dry sieving.

### EXAMPLE 2

### Preparation of Yellow Dyed Blank PLA/PGA Microparticles

Blank, yellow dyed microparticles were prepared using 50/50 PLA/PGA (BPI), and the microparticle preparation technique of solvent evaporation from Dulbecco's phosphate buffered saline solution. To 250 ml of Dulbecco's phosphate buffered saline (PBS) solution was added 0.625 g of Air Products V-205 poly(vinyl alcohol) (PVA) to prepare a 0.25% w/v solution of PVA in PBS. PVA was added to room temperature PBS and dissolution was assisted by heating to 55°C with stirring. This solution was allowed to cool to room temperature before use.

2.00 g of PLA/PGA (50/50), inherent viscosity of 0.42 dL/g in HFIP at 30°C (M_{W} = 29,000 Daltons, M_{N} = 20,000 Daltons), BPI, was dissolved in approximately 40 ml of methylene chloride. To this polymer solution, a trace amount (less than 0.1 g) of FLUOROL YELLOW 088, BASF #205671, was added to dye the polymer solution a fluorescent yellow. The polymer solution in total was added to the vortex of the PVA solution stirred at 300 RPM. Stirring was allowed to continue overnight to allow complete evaporation of methylene chloride. The resulting yellow microparticles were recovered using vacuum filtration. The microparticles were washed several times with Dulbecco's PBS, and were then placed on a drying dish in a desiccator under vacuum to dry. 1.98 g. of dried microparticles were recovered, which were sieved to the following mesh cuts (U.S. Standard):
>60 mesh (>250 microns) - trace (discarded)
60-120 mesh (250 microns - 125 microns) - 0.34 g
<120 mesh (<125 microns) - 1.64 g

### EXAMPLE 3

Dyed microparticles were used to visualize the location of the particles after in ovo injection, throughout the later periods of incubation and after hatch. 5 mg red-colored microparticles, 60-120 mesh, from Example 1 in a 100 ml 0.15 M sucrose were injected into the air cell of embryonated eggs on day E14. Three to five eggs were opened on each successive day of incubation. In addition, 3-5 chicks were necropsied on the day of hatch, as well as on each of the next three days of life. Twenty four hours after the injection, most of the microparticles had migrated between the two membranes to a location at the sharp end or caudal (bottom) of the egg opposite from the air cell. The microparticles were bathed in albumen fluid and were positioned between two thin membranes that were highly vascularized. The microparticles remained in this location throughout the incubation period.

As embryogensis approached the final day of incubation (E21) the colored particles appeared to begin to be internalized in the abdomen along with the inner membrane and yolk sac. Microscopic examination of the spherical microparticles demonstrated that they were slowly degrading throughout the week of incubation. Moreover, the shapes became irregular and larger with time, as if the microparticles had fused. Upon necropsy of the hatched chicks, the microparticles were found to be present on the peritoneum around the umbilicus. Significant amounts of intact polymer were present even after three days of life.

The fact that intact biodegradable microparticles were identified in the chick after hatch indicates that such an in ovo mode of administration will effectively release desired compound during embryonic life and during the neonatal period. The green dyed microparticles, 60-120 mesh, from Example 1 migrated similarly between the two membranes within 24 hours after injection into the air cell, however the embryos died within 48 hours from dye toxicity.

### EXAMPLE 4

Non-phospholipid liposomes were also prepared with growth hormone releasing hormone, hereinafter referred to as somatogenin, which is the name proposed for this compound to the United States Adopted Names Council (USAN). Somatogenin, as used herein, refers to a 4-methylhippuroyl (1) porcine GHRH (2-76)-OH, which is an analog of a natural porcine GHRH precursor. This compound has a molecular weight of 8846.89 daltons and its molecular formula is C₃₇₉H₆₂₄N₁₂₇O₁₁₈. Methods for the preparation of somatogenin are described in U.S. Patent Application, Serial No. 07/692,090, filed on April 26, 1991 and entitled "Superactive GRF Analogs". The structure of somatogenin is represented by the following:

*Gly.Ala.Asp.Ala.Ile.Phe.Thr.Asn.Asn.Tyr.Arg.Arg.Val.Leu.Thr.Gln.Leu.Ser.
1 5 10 15

Ala.Arg.Arg.Leu.Leu.Gln.Asp.Ile.Leu.Ser.Arg.Gln.Gln.Gly.Glu.Arg.Asn.Gln.Glu.
20 25 30 35

Gln.Gly.Ala.Arg.Val.Arg.Leu.Gly.Arg.Gln.Val.Asp.Ser.Leu.Trp.Ala.Asp.Gln.Arg.
40 45 50 55

Gln.Leu.Ala.Leu.Glu.Ser.Ile.Leu.Ala.Thr.Leu.Leu.Gln.Glu.His.Arg.Asn.Ser.Gln.
60 65 70 75

Gly.OH

* Acyl-group = pMeBenzoyl

**COMPOSITION TABLE**

| | | | | | |
|---|---|---|---|---|---|
| Ala | 7 | Gly | 5 | Pro | 0 |
| Arg | 11 | His | 1 | Ser | 5 |
| Asp | 4 | Ile | 3 | Thr | 3 |
| Asn | 4 | Leu | 12 | Tyr | 1 |
| Cys | 0 | Lys | 0 | Trp | 1 |
| Glu | 4 | Met | 0 | Val | 3 |
| Gln | 11 | Phe | 1 | | |

The liposomes were prepared in accordance with the following procedure. Negatively charged lipid vesicles were prepared using polyoxyethylene cetyl ether (0.696 g), cholesterol hemisuccinate (0.073 g), dicetyl phosphate (0.055 g) and somatogenin in 0.01 M acetic acid (10 ml @ approx. 1 mg/ml). Positively charged liposomes were prepared from the same materials, except that the dicetyl phosphate was replaced with cetyl trimethylammonium (0.036 g).

The lipid materials were first placed in a beaker and heated to approximately 80°C. The somatogenin was dissolved into 10 ml of 0.01 M CH₃COOH to achieve 1 mg/ml. 30 cc syringes and a 3-way stopcock were preheated on a hot plate to about 80°C. The somatogenin solution was also heated to about 80°C. The lipid and aqueous components were then charged to different syringes and placed at right angles on the 3-way stopcock. The lipid phase was injected into the aqueous phase by forcing it through the stopcock. The material was then forced back and forth as rapidly as possible for two minutes. The material was then transferred to vacutainer tubes, and thereafter centrifuged at 2500 rpm for 20 minutes - with no separation occurring. A small sample (about 1 ml) was centrifuged at 10,000 rpm for 15 minutes, again with no separation. The materials were combined and refrigerated for later use. The lipid vesicles were subsequently injected into the air cell of eggs, and were found to migrate between the two membranes within the egg similarly to the PLA/PGA microparticles.

### EXAMPLE 5

### Somatogenin/PLA/PGA (85/15) Microparticles

Microspheres were prepared using PLA/PGA, 85/15, BPI, and the microsphere preparation technique of solvent evaporation from Dulbecco's phosphate buffered saline (PBS) containing polyvinyl alcohol (PVA). To 250 ml of freshly prepared Dulbecco's PBS (w/o CaCl₂) was added 1.0 g of Air Products Airvol 205 polyvinyl alcohol to prepare a 0.4% w/v solution of PVA in PBS. The PVA was dispersed in PBS at room temperature using magnetic stirring, and the temperature was raised to 45°C to complete PVA dissolution. The Dulbecco's PBS solution containing PVA was allowed to return to room temperature before use.

1.80 g of PLA/PGA, 85/15, BPI, inherent viscosity of 0.58 dL g in CHCl₃ @ 30°C = (M_{W} = 90,900 Daltons, M_{N} = 50,100 Daltons), was dissolved in approximately 50 ml of methylene chloride. To this polymer solution was added 0.20 g of air milled somatogenin. The active somatogenin was dispersed to primary particle size using gentle swirling and sonication for approximately one minute. The polymer solution - somatogenin suspension was added to the vortex of the stirred Dulbecco's PBS containing PVA. The rate of stirring was 270-280 RPM. A 400 ml Pyrex beaker and plastic stirring rod with 3-bladed propeller were used to contain and stir the resulting oil-in-water emulsion. This was allowed to continue stirring overnight, resulting in complete evaporation of methylene chloride and the subsequent formation of microspheres, which were recovered using vacuum filtration. The microspheres were placed in a drying dish in a dessicater under vacuum to dry. 1.80 g + 1.85 g (3.65 g) of dried microparticles were recovered, and sieved to the following mesh cuts:
>60 mesh - trace
60-120 mesh - 2.66 g
<120 mesh - 0.94 g
Assay of combined product showed a 9.8% w/w (10.2, 9.4, 9.7) loading, as compared to 10% theoretical.

### EXAMPLE 6

### Somatogenin/PLA/PGA (85/15) Microparticles

Somatogenin containing microparticles were prepared using PLA/PGA, 85/15, BPI, and the microparticle preparation technique of solvent evaporation from Dulbecco's phosphate buffered saline (PBS) containing polyvinyl alcohol. To 250 ml of Dulbecco's PBS (w/o CaCl₂) was added 1.02 g of Air Products Airvol 205, polyvinyl alcohol to prepare a 0.4% w/v solution of PVA in PBS. The PVA was dispersed in PBS at room temperature, and the temperature raised to 45°C to facilitate dissolution. The PBS containing PVA solution was allowed to return to room temperature before use.

1.75 g of PLA/PGA, 85/15, BPI, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.25 g of air milled somatogenin. The somatogenin was dispersed to primary particle size using gentle agitation and sonication for approximately one minute. The polymer solution somatogenin suspension was added to the vortex of the stirred Dulbecco's PBS containing PVA, and stirring was continued overnight, resulting in complete evaporation of methylene chloride. The resulting microparticles were recovered using vacuum filtration and placed in a drying dish in a dessicater under vacuum to dry, yielding 1.81 g of dried microparticles, sieved to the following mesh cuts (U.S. Standard):
>60 mesh - none
60-120 mesh - 0.96 g
<120 mesh - 0.08 g
Assay of the 60-120 mesh cut showed 12.9% w/w somatogenin (12.5% theoretical).

### EXAMPLE 7

### Somatogenin/PLA/PGA (85/15) Microparticles

Somatogenin containing microparticles were prepared using PLA/PGA, 85/15, BPI, and the microparticle preparation technique of solvent evaporation from Dulbecco's phosphate buffered saline (PBS) containing polyvinyl alcohol (PVA). To 250 ml of Dulbecco's PBS (w/o CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.52 g of PLA/PGA, 85/15, BPI, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.50 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a dessicater under vacuum to dry. 1.80 g of dried microparticles were recovered and sieved to the following mesh cuts (U.S. Standard):
>60 mesh - none
60-120 mesh - 1.30 g
<120 mesh - 0.17 g
Assay of the 60-120 mesh cut showed 24.9% w/w somatogenin loading (25% theoretical).

### EXAMPLE 8

### Somatogenin/PLA/PGA (85/15) Microparticles

Microparticles containing somatogenin were prepared using PLA/PGA (85/15) BPI and the microparticle preparation technique of solvent evaporation from Dulbecco's PBS containing polyvinyl alcohol (PVA). To 250 ml of freshly prepared Dulbecco's phosphate buffered saline (PBS) (w/o CaCl₂), was added 1.0 g of Air Products V-205 PVA to prepare a 0.4% w/v solution of PVA in PBS. The PVA was dispersed in the PBS at room temperature, and the temperature was raised to 45°C to aid dissolution. This solution was allowed to cool to room temperature before use. This solution contained 0.4% w/v PVA in PBS.

1.5 g of PLA/PGA, 85/15, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.5 g of micronized somatogenin. (All glassware and stirrer/shaft were rinsed in acetonitrile before allowing them to come in contact with somatogenin.) The somatogenin was dispersed to primary particle size using gentle agitation and sonication for approximately one minute (at room temperature). The polymer solution/somatogenin suspension was added to the vortex of the stirred Dulbecco's PBS solution containing PVA. The stirring was allowed to continue overnight, allowing the dichloromethane solvent to completely evaporate. The microparticles were recovered using vacuum filtration and were placed in a drying dish in a desiccator under vacuum to dry. 1.84 g of dried microparticles were recovered and were sieved to the following mesh cuts (U.S. Standard):
>60 mesh - 0.01 g (discarded)
60-120 mesh - 0.86 g
<120 mesh - 0.94 g
Repetition of the foregoing process yielded 1.87 g of dried microparticles, which were sieved to the following mesh cuts (U.S. Standard):
>60 mesh - trace (discarded)
60-120 mesh - 1.10 g
<120 mesh - 0.71 g
These particles assayed as follows:
60-120 mesh = 15.7 ± 2.1% loading (somatogenin)
<120 mesh = 15.2 ± 0.8% w/w loading (somatogenin)

### EXAMPLE 9

Test materials for in ovo injection studies, using the products of Example 8, were prepared as follows. The following treatment (Trt) materials were prepared:
Trt A: water as control;
Trt B: micronized somatogenin dispersed in 50 ml purified water to yield a concentration of 60mg somatogenin per 100ml water;
Trt C: micronized somatogenin dispersed in 50 ml purified water to yield a concentration of 600mg somatogenin per 100ml water;
Trt D: microparticles (Somatogenin PLA/PGA, 60-120 mesh) dispersed in 50 ml of 2% w/v sodium carboxy methyl cellulose (NaCMC 330) to yield a suspension containing microparticles equivalent to 60mg somatogenin/100ml (amount dispersed on loading);
Trt E: microparticles (Somatogenin PLA/PGA, 60-120 mesh) dispersed in 50 ml of 2% w/v NaCMC 330 to yield a suspension containing microparticles equivalent to 600mg somatogenin/100ml (amount dispersed based on loading); and
Trt F: blank microparticles (PLA/PGA, 60-120 mesh) dispersed in 50 ml of 2% w/v NaCMC 330, approximately equivalent to high dose somatogenin PLA/PGA (Trt E).
Upon injection of the foregoing materials into the air cells of eggs, the particles migrated to the bottoms of the eggs, typically within a few hours. No decrease in hatchability was noted for any treatment.

### EXAMPLE 10

10% w/w somatogenin microspheres were prepared by dissolving 1.8 g of 85/15 poly(lactide-co-glycolide), inherent viscosity of 0.58 dL/g in CHCl₃ @ 30°C (M_{W} = 90,900 Daltons, M_{N} = 50,100 Daltons), Birmingham Polymers, Inc., in approximately 50 ml of methylene chloride, and adding 0.2 g of micronized somatogenin. The polymer solution/somatogenin suspension was emulsified into phosphate buffered saline containing poly(vinyl alcohol). The oil in water emulsion was stirred for approximately 16 hours, allowing complete solvent evaporation and the subsequent formation of microspheres. Microspheres were collected by vacuum filtration, dried in a desiccator under vacuum, and sieved to the size.

### EXAMPLE 11

The microparticles prepared in accordance with Example 10 were used to demonstrate the time release aspect of containment of the somatogenin. Studies were conducted in vitro, using perifusion with 0.1 N acetic acid at a flow rate of 200 ml/min. The results of these studies are shown in FIGS. 1 and 2. These results demonstrate that the microparticles release the contained agent over time, and further that the rate of release is controlled by the percentage of loading of the agent.

While the invention has been illustrated and described in detail in the foregoing description, the same is to be considered as illustrative and not restrictive in character, it being understood that only the preferred embodiment has been shown and described and that all changes and modifications that come within the spirit of the invention are desired to be protected.

In another aspect of the present invention, there is provided a composition including biocompatible, biodegradable microparticles having a polyester matrix and from about 5% to about 25% by weight of a biologically active, water-soluble polypeptide dispersed throughout the matrix. In another aspect of the invention, microparticles containing biologically active peptides are prepared by dissolving polyester in an organic solvent; suspending a biologically active agent in the polyester solution; emulsifying the suspension into an aqueous medium in which the agent is insoluble and evaporating the solvent from the emulsion to produce microparticles. One advantage of the present invention is the ability to conveniently prepare microparticles which have a relatively high ratio of peptide to polyester. In a still further aspect, there is provided a method for administering a bioactive agent to an organism which involves suspending the microparticles in a suitable liquid and injecting the organism.

It is an object of the present invention to provide microparticles incorporating biologically active agents, e.g., peptides, in a biodegradable and biocompatible matrix.

A further object of the present invention is to provide methods for the preparation of microparticles which contain peptide agents, which methods achieve up to 25% loading of the agents.

It is another object of the present invention to provide microparticles which contain peptide agents which are released under in vivo conditions at sustained, predictable rates.

A further object of the present invention is to provide methods for the preparation of microparticles containing peptide agents, which methods do not result in the destabilization, destruction or inactivation of the peptides.

It is another object of the present invention to provide microparticles, and methods for the preparation thereof, which permit modification of the release characteristics of the microparticles by adjustment of the matrix properties.

These and other objects, advantages and features are accomplished according to the compositions and methods of the following description of the preferred embodiment of the present invention.

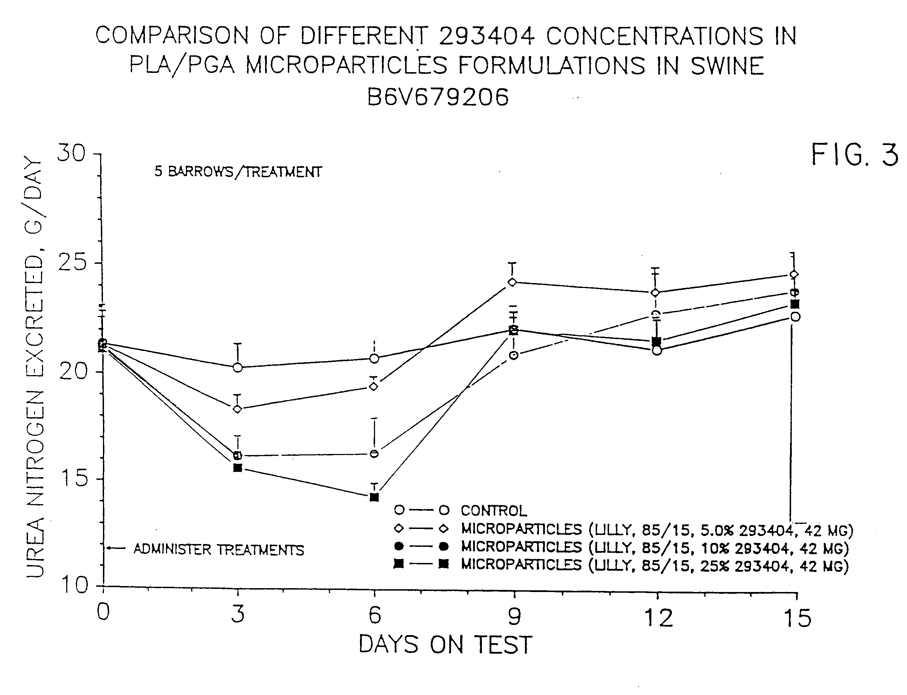

FIGS. 3-6 are graphs representing in vivo studies in swine demonstrating biological consequences of somatogenin controlled release from microparticles.

For the purposes of promoting an understanding of the principles of the invention, reference will now be made to the preferred embodiments thereof, and specific language will be used to describe the same. It will nevertheless be understood that no limitation of the scope of the invention is thereby intended, such alterations, modifications, and further applications of the principles of the invention being contemplated as would normally occur to one skilled in the art to which the invention relates.

The present invention provides microparticle formulations which comprise polyesters as the supporting matrix and peptides as the contained, biologically active agents. The microparticles are prepared by a solvent evaporation technique which incorporates the peptides in a stable and active form. The microparticles are biodegradable, and afford a controlled, sustained release of the peptides. The rate of drug release is controlled by adjusting factors such as the degree of peptide loading, molecular weight of the biodegradable polymer, and in certain instances the ratio of copolymer components. The microparticles, and preparatory methods, of the present invention are distinctive from the prior art in providing formulations in which the peptides remain chemically stable, physically stable (conformation), and biologically active. The methods of the present invention also achieve a greater proportion of peptide to polymer than prior art solvent evaporation techniques for microparticles incorporating water-soluble polypeptides.

The microparticles of the present invention provide sustained release of the contained peptides. As a result of the method of preparing the microparticles, the biologically active compounds, e.g., peptides and the like, are entrapped in the polymer network during fabrication. The peptides are then released over time during the degradation of the matrix, and to a limited extent, by diffusion through the polymer network.

The release profile may be adjusted by appropriate choice or control of various parameters. For example, release characteristics will change with the polymer composition, particularly the type and proportion of polymers; the molecular weight of the polymers; the weight-average molecular weight (M_{w}); the molecular weight range (or polydispersity) measured by the ratio of the weight-average molecular weight (M_{w}) to the number-average molecular weight (Mₙ), i.e. M_{w}/Mₙ; the size and shape of the microparticles; and the proportion of peptide to polymer, i.e., the loading.

The microparticles of the present invention may be prepared from polyesters, e.g., poly(D,L-lactide), poly(D,L-lactide-co-glycolide), poly(epsilon-caprolactone), polyaminoacids, poly(orthoesters), polyanhydrides, polyalkyl cyanoacrylates. Any polyesters which are biodegradable and/or bioerodible, and yield biocompatible materials upon degradation are contemplated.

The polylactides are especially preferred for use in accordance with the present invention. Polymeric materials for preparation of the microparticles are available commercially. The term polylactide is used in a generic sense to include polymers and mixtures of polymers of lactic acid alone, copolymers of lactic acid and glycolic acid and mixtures of such copolymers, and mixtures of such polymers and copolymers, the lactic acid being either in racemic or in optically active form. The term PLA/PGA is used herein to refer to the various copolymers of lactic and glycolic acids. The molecular weight of the polymers ranges from about 29,000 Daltons to about 90,900 Daltons. The ratio of lactide units to glycolide units ranges from 100:0 to 50:50. For example, one PLA/PGA polymer used had a ratio of PLA to PGA of 85:15, an inherent viscosity of 0.58 dL/g, and a molecular weight of approximately 90,900 daltons. This particular polymer was obtained from Birmingham Polymers, Incorporate (BPI), and is prepared by ionic, ring-opening, addition polymerization of the respective cyclic dimers of lactic and glycolic acids.

Upon exposure to water, the polyesters degrade through hydrolysis of ester linkages to yield biocompatible products. For example, the preferred PLA/PGA polymers degrade to lactic acid and glycolic acid. The 85:15 PLA/PGA copolymer has been reported to have a biodegradation time of approximately 5 months, depending on surface area, porosity and molecular weight. Other blends of PLA/PGA have degradation times ranging from 2 months to 24 months. A 50:50 PLA/PGA polymer, which has alternately been used, has a reported degradation time of 2 months. Similarly, the other polyesters used in the present invention have suitable biocompatibility and degradation times.

The microparticles prepared in accordance with the disclosed methods are suitable for use in the administration of various peptide agents. An advantage of this invention is that it provides microparticles containing small, biologically-active polypeptides. Generally, peptides of molecular weights up to 10,000 daltons are called polypeptides, while peptides of molecular weights above 10,000 daltons are called proteins. In the past, the solvent evaporation technique for preparing microparticles has typically resulted in low loading of small water-soluble peptides due to the incompatibility of such peptides with this process. Typically, loadings of below 10% have been reported. Low loading of microparticles may not provide a proper dose. However, in spite of its limitations, the solvent evaporation procedure is preferred because of its ease and reliability. The methods of the present invention employ an aqueous medium in which the biological agent is insoluble, and the process results in microparticles having loading up to 25%. The present invention addresses the need for convenient methods to incorporate polypeptides into microparticles with loading of 5-25%.

The preparatory method of the present invention is demonstrated in the following examples. In general, the polymeric material is dissolved in a suitable organic solvent, e.g. methylene chloride or chloroform, and the bioactive, water-soluble peptide, e.g. somatogenin, is added thereto. Preferably, the somatogenin has been reduced in particle size by air milling. See U.S. Patent No. 5,021,554, the pertinent portions of which are hereby incorporated by reference. The polymer solution/peptide suspension is preferably sonicated to primary particles size. The suspension is then emulsified into a suitable aqueous media in which the bioactive peptide is insoluble, preferably, phosphate buffered saline. The medium may contain a stabilizer such as poly(vinyl alcohol), sodium dodecyl sulfate, cetyltrimethyl ammonium bromide, methylcellulose or gelatin. Various excipients may be added to the above at concentrations up to about 10%. Excipients may include, for example, fatty acids, such as stearic acid, myristic acid, lauric acid, and preferably, palmitic acid. The solvent is allowed to evaporate with the consequent formation of the microparticles. The evaporation is preferably aided with stirring. The particles are collected, preferably by vacuum filtration. The particles may then be dried in a desiccator under vacuum, and sieved to size appropriate for use.

This invention is applicable to polypeptides, and the following list, which is not intended to be exhaustive, is indicative of polypeptides which may be employed in the formulations of this invention: growth horone releasing factor, somatogenin, oxytocin, vasopressin, adrenocorticotrophic hormone (ACTH), epidermal growth factor (EGF), prolactin, luliberin or luteininzing hormone releasing hormone (LH-RH), transforming growth factor, insulin, somatostatin, glucagon, interferon, gastrin, tetragastrin, pentagastrin, urogastrone, secretin, calcitonin, enkephalins, endorphins, angiotensins, renin, bradykinin, bacitracins, polymyxins, colistins, tyrocidin, gramicidines, and synthetic analogs and modifications and pharmacologically-active fragments thereof.

Upon collection of the microparticles, a desired range of particle sizes may be directly obtained by sieving. In addition, the particles may be milled, such as by an ultracentrifuge mill. The microparticles are preferably sized less than about 250 microns, more preferably between about 120 and about 250 microns.

The resulting particles are combined with a suitable liquid for administration. The microparticles may be dispersed, e.g. suspended, in other known, suitable liquid vehicles for administration, such as water, dextrose solution, glycerol, or water containing 2% w/v sodium carboxyl-methyl-cellulose 330 (NaCMC) or hydroxypropylmethyl cellulose (HPMC), to increase viscosity to prevent microparticles from settling out of suspension. The particles may be administered to living organisms; including pigs or other mammals and birds, such as chickens or turkeys. For example, somatogenin microparticle formulations may be placed in suspension and injected subcutaneously in the flank of swine. The microparticle formulations may also be injected directly into a bird or administered indirectly into a bird embryo by injection into the air sac of an egg as described hereinabove.

It will further be appreciated by those skilled in the art that the microparticles containing incorporated drugs for release to target cells or tissues may be administered alone or in a mixture with appropriate pharmaceutical diluents, carriers, excipients or adjuvants suitably selected with respect to the intended route of administration and conventional pharmaceutical practices. These inert pharmaceutically acceptable adjuvants are well known in the art. For example, for parenteral injections, dosage unit forms may be utilized to accomplish intravenous, intramuscular or subcutaneous administration, and for such parenteral administration, suitable sterile aqueous or non-aqueous solutions or suspensions, optionally containing appropriate solutes to effect isotonicity, will be employed.

The following specific examples are provided for purposes of illustrating the invention, and no limitations on the invention are intended thereby.

### EXAMPLE 12

### 10% Somatogenin in PLA/PGA (85/15) Microparticles

Microparticles were prepared using PLA/PGA, 85/15, BPI, and the microparticle preparation technique of solvent evaporation from Dulbecco's phosphate buffered saline (PBS) containing polyvinyl alcohol (PVA). To 250 ml of freshly prepared Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature using magnetic stirring, and the temperature was raised to 45°C to complete PVA dissolution. The Dulbecco's PBS solution containing PVA was allowed to return to room temperature before use.

1.80 g of PLA/PGA, 85/15, BPI, inherent viscosity of 0.58 dL g in CHCl₃ @ 30°C (M_{W} = 90,900 Daltons, M_{N} = 50,100 Daltons), was dissolved in approximately 50 ml of methylene chloride. To this polymer solution was added 0.20 g of air milled somatogenin. The active somatogenin was dispersed to primary particle size using gentle swirling and sonication for approximately one minute. The polymer solution - somatogenin suspension was added to the vortex of the stirred Dulbecco's PBS containing PVA. The rate of stirring was 270-280 RPM. A 400 ml Pyrex beaker and plastic stirring rod with 3-bladed propeller were used to contain and stir the resulting oil-in-water emulsion. This was allowed to continue stirring overnight, resulting in complete evaporation of methylene chloride and the subsequent formation of microspheres, which were recovered using vacuum filtration. The microspheres were placed in a drying dish in a desicator under vacuum to dry. 1.80 g + 1.85 g (3.65 g, representing two batches) of dried microparticles were recovered, and sieved to the following mesh cuts:
>60 mesh - trace
60-120 mesh - 2.66 g
<120 mesh - 0.94 g
Assay of combined product showed a 9.8% w/w (10.2, 9.4, 9.7) loading, as compared to 10% theoretical.

### EXAMPLE 13

### 12.5% Somatogenin in PLA/PGA (85/15) Microparticles

Somatogenin containing microparticles were prepared using PLA/PGA, 85/15, BPI, and the microparticle preparation technique of solvent evaporation from Dulbecco's phosphate buffered saline (PBS) containing polyvinyl alcohol. To 250 ml of Dulbecco's PBS (without CaCl₂) was added 1.02 g of Air Products Airvol 205, polyvinyl alcohol to prepare a 0.4% w/v solution of PVA in PBS. The PVA was dispersed in PBS at room temperature, and the temperature raised to 45°C to facilitate dissolution. The PBS containing PVA solution was allowed to return to room temperature before use.

1.75 g of PLA/PGA, 85/15, BPI, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.25 g of air milled somatogenin. The somatogenin was dispersed to primary particle size using gentle agitation and sonication for approximately one minute. The polymer solution somatogenin suspension was added to the vortex of the stirred Dulbecco's PBS containing PVA, and stirring was continued overnight, resulting in complete evaporation of methylene chloride. The resulting microparticles were recovered using vacuum filtration and placed in a drying dish in a desicator under vacuum to dry, yielding 1.81 g of dried microparticles, sieved to the following mesh cuts (U.S. Standard):
>60 mesh - none
60-120 mesh - 0.96 g
<120 mesh - 0.08 g
Assay of the 60-120 mesh cut showed 12.9% w/w somatogenin (12.5% theoretical).

### EXAMPLE 14

### 15% Somatogenin in PLA/PGA (85/15) Microparticles

Microparticles containing somatogenin were prepared using PLA/PGA (85/15) BPI and the microparticle preparation technique of solvent evaporation from Dulbecco's PBS containing polyvinyl alcohol (PVA). To 250 ml of freshly prepared Dulbecco's phosphate buffered saline (PBS) (without CaCl₂), was added 1.0 g of Air Products V-205 PVA to prepare a 0.4% w/v solution of PVA in PBS. The PVA was dispersed in the PBS at room temperature, and the temperature was raised to 45°C to aid dissolution. This solution was allowed to cool to room temperature before use. This solution contained 0.4% w/v PVA in PBS.

1.5 g of PLA/PGA, 85/15, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.5 g of micronized somatogenin. The somatogenin was dispersed to primary particle size using gentle agitation and sonication for approximately one minute (at room temperature). The polymer solution/somatogenin suspension was added to the vortex of the stirred Dulbecco's PBS solution containing PVA. The stirring was allowed to continue overnight, allowing the methylene chloride solvent to completely evaporate. The microparticles were recovered using vacuum filtration and were placed in a drying dish in a desiccator under vacuum to dry. 1.84 g of dried microparticles were recovered and were sieved to the following mesh cuts (U.S. Standard):
>60 mesh - 0.01 g (discarded)
60-120 mesh - 0.86 g
<120 mesh - 0.94 g
Repetition of the foregoing process yielded 1.87 g of dried microparticles, which were sieved to the following mesh cuts (U.S. Standard):
>60 mesh - trace (discarded)
60-120 mesh - 1.10 g
<120 mesh - 0.71 g
These particles assayed as follows:
60-120 mesh = 15.7 ± 2.1% loading (somatogenin)
<120 mesh = 15.2 ± 0.8% w/w loading (somatogenin)

### EXAMPLE 15

### 25% Somatogenin in PLA/PGA (85/15) Microparticles

Somatogenin containing microparticles were prepared using PLA/PGA, 85/15, BPI, and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.52 g of PLA/PGA, 85/15, BPI, inherent viscosity of 0.58 dL/g in CHCl₃ @ 30°C (M_{W} = 90,900 Daltons, M_{N} = 50,100 Daltons), was dissolved in approximately 50 ml of methylene chloride. To this polymer solution was added 0.50 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.80 g of dried microparticles were recovered and sieved to the following mesh cuts (U.S. Standard):
>60 mesh - none
60-120 mesh - 1.30 g
<120 mesh - 0.17 g
Assay of the 60-120 mesh cut showed 24.9% w/w somatogenin loading (25% theoretical).

### EXAMPLE 16

### 25% Somatogenin in PLA/PGA (50/50) Microparticles

Microparticles were prepared using PLA/PGA, 50/50, BPI, and the microparticle preparation technique of solvent evaporation from Dulbecco's phosphate buffered saline (PBS) containing polyvinyl alcohol (PVA). To 250 ml of freshly prepared Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature using magnetic stirring, and the temperature was raised to 45°C to complete PVA dissolution. The Dulbecco's PBS solution containing PVA was allowed to return to room temperature before use.

1.50 g of PLA/PGA, 50/50, BPI, inherent viscosity of 0.42 dL g in CHCl₃ @ 30°C (M_{W} = 29,000 Daltons, M_{N} = 20,000 Daltons), was dissolved in approximately 50 ml of methylene chloride. To this polymer solution was added 0.50 g of air milled somatogenin. The active somatogenin was dispersed to primary particle size using gentle swirling and sonication for approximately one minute. The polymer solution - somatogenin suspension was added to the vortex of the stirred Dulbecco's PBS containing PVA. The rate of stirring was 270-280 RPM. A 400 ml Pyrex beaker and plastic stirring rod with 3-bladed propeller were used to contain and stir the resulting oil-in-water emulsion. This was allowed to continue stirring overnight, resulting in complete evaporation of methylene chloride and the subsequent formation of microspheres, which were recovered using vacuum filtration. The microspheres were placed in a drying dish in a desicator under vacuum to dry. 1.92 g of dried microparticles were recovered, and sieved to the following mesh cuts:
>60 mesh - trace (discard)
60-120 mesh - 1.11 g
<120 mesh - 0.77 g

### EXAMPLE 17

### 10% Somatogenin in Poly(DL-lactide) Microparticles

Somatogenin containing microparticles were prepared using poly (DL-lactide) PLA/PGA, 85/15, BPI, and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.80 g of poly (DL-lactide), BPI, inherent viscosity of 0.34 dL/g in CHCl₃ @ 30°C (M_{W} = 38,300 Daltons, M_{N} = 24,300 Daltons), was dissolved in approximately 50 ml of methylene chloride. To this polymer solution was added 0.20 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.68 g of dried microparticles were recovered and sieved to the following mesh cuts (U.S. Standard):
>60 mesh - 0.02 g
60-120 mesh - 1.36 g
<120 mesh - 0.18 g

### EXAMPLE 18

### 10% Somatogenin in Poly(caprolactone) Microparticles

Somatogenin containing microparticles were prepared using poly(caprolactone), BPI, and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.80 g of poly(caprolactone) BPI, inherent viscosity of 1.27 dL/g in CHCl₃ @ 30°C [(M_{W} = 143,000 Daltons, M_{N} = 83,000 Daltons),] was dissolved in approximately 50 ml of methylene chloride. To this polymer solution was added 0.50 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.85 g of dried microparticles were recovered and sieved to the following mesh cuts (U.S. Standard):
>60 mesh - trace
60-120 mesh - 1.27 g
<120 mesh - 0.58 g

### EXAMPLE 19

### 10% Somatogenin in Poly(caprolactone)-Palmitic Acid (1%) Microparticles

Somatogenin containing microparticles were prepared using poly(caprolactone) and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.78 g of polycaprolactone, low molecular weight, Polysciences, was dissolved in approximately 50 ml of methylene chloride. To this polymer solution was added 0.02 g of palmitic acid. To this solution of polymer-fatty acid was added 0.20 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.89 g of dried microparticles were recovered and sieved to the following mesh cuts (U.S. Standard):
>60 mesh - 0.03 g
60-120 mesh - 1.15 g
<120 mesh - 0.74 g

### EXAMPLE 20

### 10% Somatogenin in Poly(caprolactone)-Palmitic acid (5%) Microparticles

Somatogenin containing microparticles were prepared using poly(caprolactone), and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.70 g of poly(caprolactone), low molecular weight, Polysciences, was dissolved in approximately 50 ml of methylene chloride. To this polymer solution was added 0.10 g of palmitic acid. To this polymer-fatty acid solution was added 0.20 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.93 g of dried microparticles were recovered and sieved to the following mesh cuts (U.S. Standard):
>60 mesh - 0.01 g
60-120 mesh - 1.06 g
<120 mesh - 0.82 g

### EXAMPLE 21

### 10% Somatogenin in Poly(caprolactone)-Palmitic acid 10% Microparticles

Somatogenin containing microparticles were prepared using poly(caprolactone), and the microparticle preparation technique of solvent evaporation from Dulbecco's phosphate buffered saline (PBS) containing polyvinyl alcohol (PVA). To 250 ml of Dulbecco's PBS (without CaCl₂) was added 1.0 g of Air Products Airvol 205 polyvinyl alcohol to prepare a 0.4% w/v solution of PVA in PBS. The PVA was dispersed in PBS at room temperature and the temperature raised to 45°C to aid dissolution. This PVA containing solution was returned to room temperature before use.

1.60 g of poly(caprolactone), low molecular weight, Polysciences was dissolved in approximately 50 ml of methylene chloride. To this polymer solution was added 0.20 g of palmitic acid. To this solution of polymer-fatty acid was added 0.20 g of air milled somatogenin, and the active somatogenin was dispersed to primary particle size using gentle agitation and sonication for approximately one minute. The polymer solution-somatogenin suspension was added to the vortex of the stirred Dulbecco's PBS containing PVA. A 400 ml. Pyrex beaker and a plastic 3-bladed stirrer and shaft were used to contain and agitate the oil-in-water emulsion. Stirring continued overnight, resulting in complete evaporation of methylene chloride. The resulting microparticles were recovered using vacuum filtration and placed in a drying dish in a desicator under vacuum to dry. 1.91 g of dried microparticles were recovered and sieved to the following mesh cuts (U.S. Standard):
>60 mesh - trace
60-120 mesh - 1.17 g
<120 mesh - 0.69 g

### EXAMPLE 22

### Effect of Somatogenin Concentration on Payout Profile

The microparticles prepared in accordance with the previous examples were used to determine the effect of somatogenin (LY293404) concentration in different PLA/PGA microparticle concentrations. Payout characteristics were measured by urinary urea nitrogen excretion and serum growth hormone levels in finishing swine. The microparticle formulations were placed in suspension, using 2% W/V sodium carboxyl-methyl-cellulose 330 (NaCMC) in Nano-pure II purified water. The suspension was injected subcutaneously in the flank. The following treatment (Trt) materials were prepared:
- Trt A:: water as control;
- Trt B:: 5% Somatogenin PLA/PGA (85:15) microparticles (60-120 mesh, MW = 90,900, Viscosity = 0.58 dL/g in CHCl3 @ 30 C) suspended in water containing 2% w/v NaCMC 330 to give a dose of 42 mg somatogenin/animal.
- Trt C:: 10% Somatogenin PLA/PLG (85:15) microparticles (60-120 mesh, MW = 90,900, Viscosity = 0.58 dL/g in CHCl₃ @ 30 C) suspended in water containing 2% w/v NaCMC 330 to give a dose of 42 mg somatogenin/animal.
- Trt D:: 25% Somatogenin PLA/PLG (85:15) microparticles (60-120 mesh, MW = 90,900, Viscosity = 0.58 dL/g in CHCl₃ @ 30 C) suspended in water containing 2% w/v NaCMC 330 to give a dose of 42 mg somatogenin/animal.

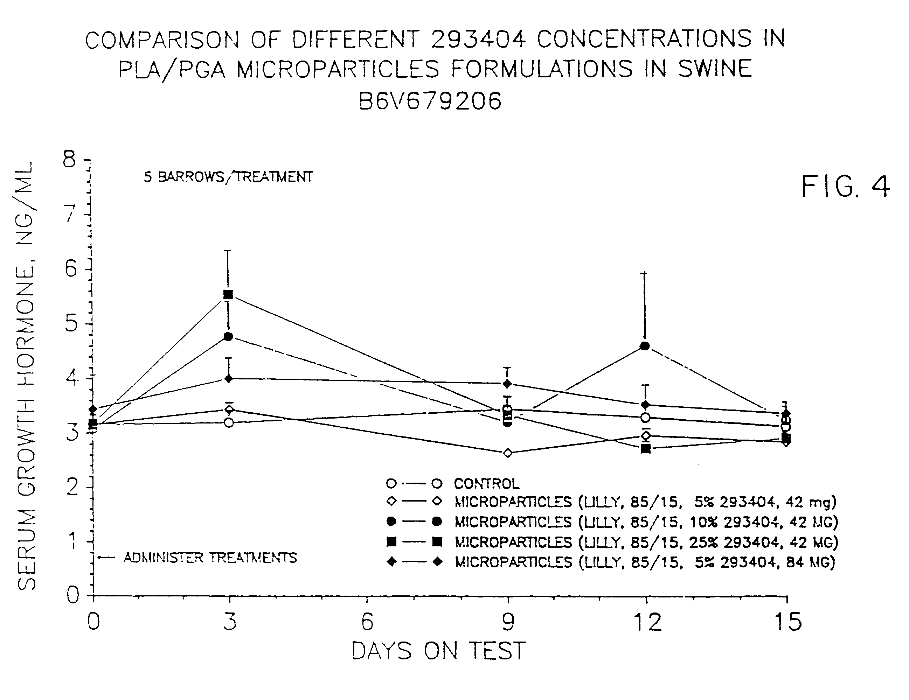

The results of this study demonstrate the efficacy of the microparticles for prolonged release of a contained peptide. Urinary nitrogen reduction is a measurement of growth hormone which is stimulated by somatogenin release from microparticles. As shown in FIG. 3, there was a reduction in urinary urea nitrogen excreted in all microparticle treated pigs during the first six days of treatment. The reduction was the greatest for the highest loaded (25%) microparticles, and lowest for the lowest (5%) loaded microparticles. FIG. 4 shows the direct measurement of serum growth hormone for microparticle treated pigs, further evidencing the sustained release from the microparticles of the somatogenin over time. The vertical bars in the figures represent the relative standard deviations.

### EXAMPLE 24

### Payout Profiles of Somatogenin with Different PLA/PGA Microparticle Formulations

The microparticles prepared in accordance with the previous examples were used to determine the payout characteristics of somatogenin (LY293404) when administered in different PLA/PLG microparticle formulations as measured by urinary urea nitrogen excretion and serum growth hormone levels in finishing swine. The microparticle formulations were placed in suspension, using 2% W/V sodium carboxylmethyl-cellulose 330 (NaCMC) in Nano-pure II purified water. The suspension was injected subcutaneously in the flank. The following treatment (Trt) materials were prepared:
- Trt A:: water as control;
- Trt B:: Somatogenin (3 micrograms/kg/day injected twice per day) as positive control
- Trt C:: 11.7% Somatogenin PLA/PLG (50:50) microparticles (60-120 mesh, MW = 60,700) suspended in water containing 2% w/v NaCMC 330 to give a dose of 22.68 mg somatogenin/animal.
- Trt D:: 11.4% Somatogenin PLA/PLG (50:50) microparticles (60-120 mesh, MW = 29,000) suspended in water containing 2% w/v NaCMC 330 to give a dose of 7.56 mg somatogenin/animal.
- Trt E:: 11.4% Somatogenin PLA/PLG (50:50) microparticles (60-120 mesh, MW = 29,000) suspended in water containing 2% w/v NaCMC 330 to give a dose of 22.68 mg somatogenin/animal.
- Trt F:: 13.6% Somatogenin PLA/PLG (85:15) microparticles (60-120 mesh, MW = 90,900) suspended in water containing 2% w/v NaCMC 330 to give a dose of 7.56 mg somatogenin/animal.
- Trt G:: 13.6% Somatogenin PLA/PLG (85:15) microparticles (60-120 mesh, MW = 90,900) suspended in water containing 2% w/v NaCMC 330 to give a dose of 22.68 mg somatogenin/animal.

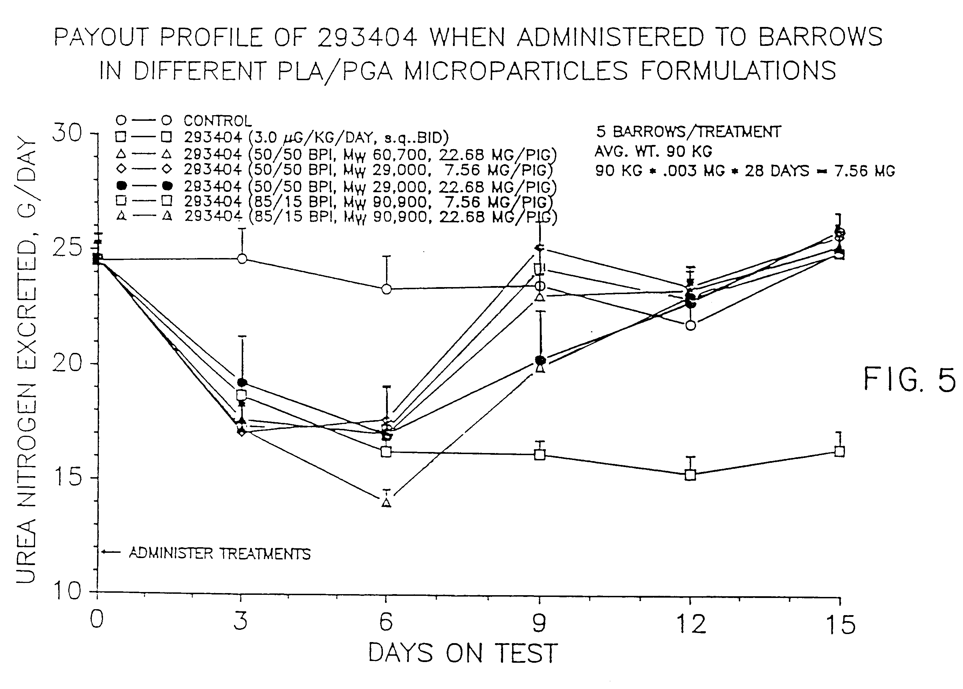

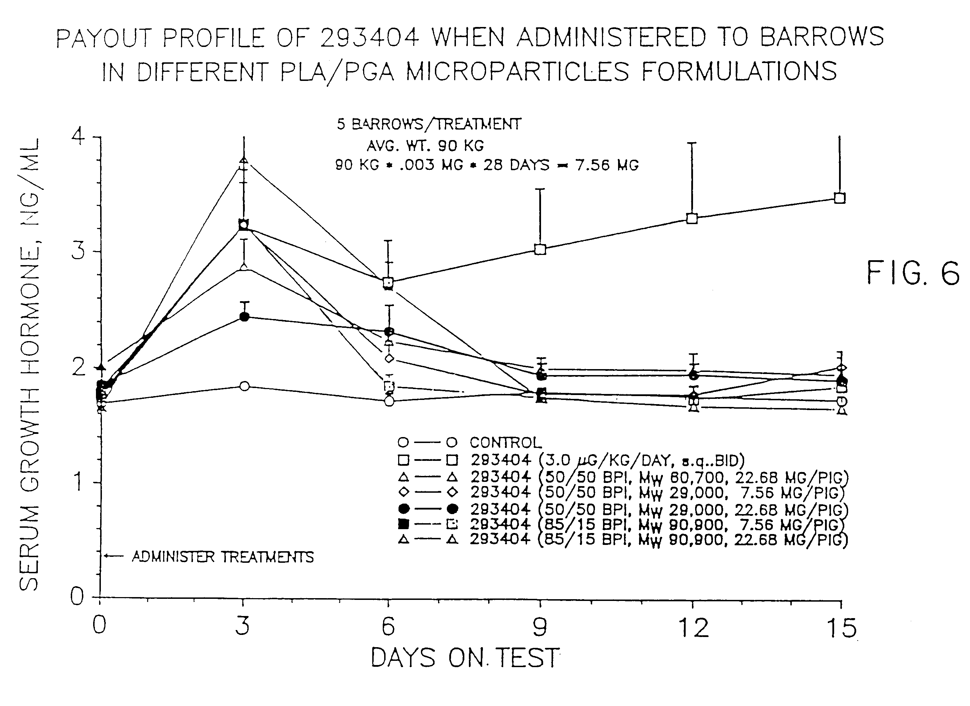

As shown in FIGS. 5 and 6, there was a reduction in urinary urea excretion and an elevation in serum GH levels in the somatogenin injected (BID) barrows at all time periods for 15 days. Urinary urea nitrogen excretion was less and serum GH levels were elevated in all microparticle treated pigs during the first 6 days of treatment. Urinary urea nitrogen excretion and serum GH levels returned to baseline levels at approximately 9 days after treatment.

While the invention has been described in detail in the foregoing description, the same is to be considered as illustrative and not restrictive in character, it being understood that only the preferred embodiments have been shown and described, and that all changes and modifications that come within the spirit of the invention are desired to be protected.

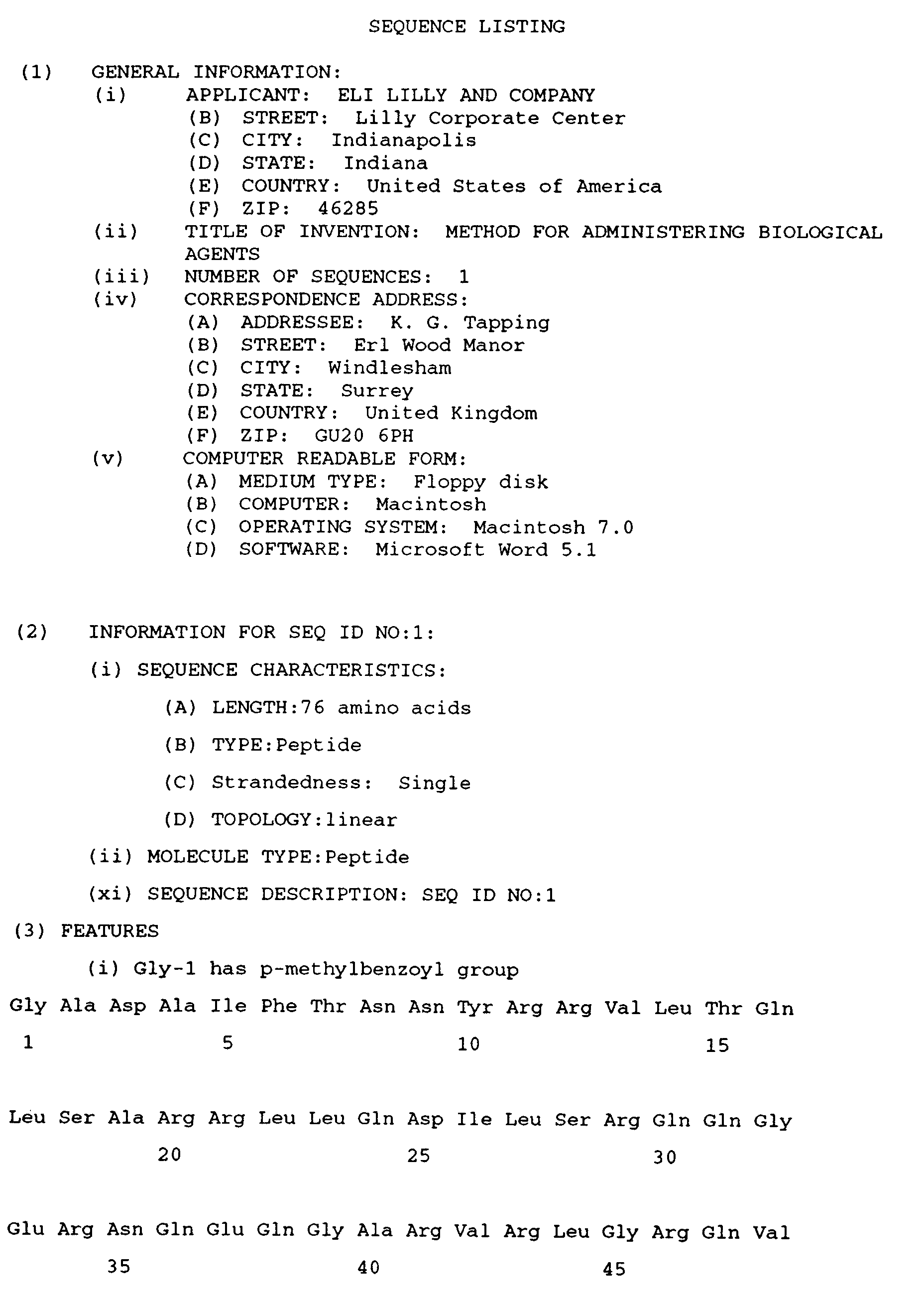

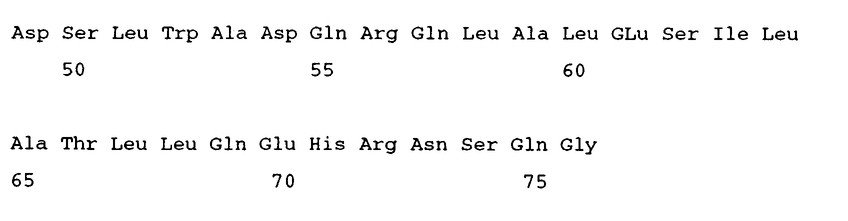

## Claims

1. The use of microparticles for the manufacture of an injectable formulation adapted for injection into an egg including an outer membrane and an inner membrane with fluid therebetween except at one end of the egg where the membranes define an air cell, said microparticles being of a size and density to migrate through the fluid between the membranes to the end of the egg opposite to the air cell.

2. The use of Claim 1 in which the microparticle is adapted to be injected into the air cell of an egg positioned with the air cell at the top, and which has a density at least as dense as the fluid between the membranes.

3. The use of claim 1 or 2 in which the microparticle has an average size less than about 500 microns.

4. The use of any of claims 1-3 for delivering a biologically active agent selected from the group consisting of growth hormone releasing factor, synthetic analogs of growth hormone releasing factor, and pharmacologically active fragments thereof.

5. The use of any of claims 1-4 wherein the microparticle comprises a polymer selected from the group consisting of polylactide polymers, polyglycolide polymers, and copolymers of lactic acid and glycolic acid.

6. A method for preparing a biodegradable, polyester microparticle containing a biologically active agent which comprises:
a. dissolving the polyester in an organic solvent;
b. adding a water-soluble, biologically active agent to the solution of step (a) to form a suspension;
c. emulsifying the suspension of step (b) into an aqueous medium in which the agent is insoluble;
d. evaporating the solvent from the emulsion to produce the microparticle; and
e. collecting the microparticle.

7. A biodegradable, polyester microparticle composition prepared by the process of claim 6.

8. The composition of claim 7 wherein the biologically active agent is selected from the group consisting of growth hormone releasing factor, synthetic analogs of growth hormone releasing factor, and pharmacologically active fragments thereof.

9. The composition of claim 7 or 8 wherein the polyester is a polylactide selected from the group consisting of a polymer of lactic acid alone, a copolymer of lactic acid and glycolic acid, a mixture of such polymers, a mixture of such copolymers, or a mixture of such polymers and copolymers.
